# NEW EUROPEAN PATENT SPECIFICATION

(11) **EP 2 479 194 B2**
(45) Date of publication and mention of the opposition decision: **01.08.2018**
(45) Mention of the grant of the patent: 28.01.2015
(21) Application number: 10817410.3
(22) Date of filing: 15.09.2010
(51) Int. Cl.: C08B 37/08, A61K 8/73, A61K 31/728, C08K 3/00, C08K 3/04, C08B 37/00

(54) **METHOD FOR MANUFACTURING LOW MOLECULAR WEIGHT HYALURONIC ACID**
VERFAHREN ZUR HERSTELLUNG VON HYALURONSÄURE MIT GERINGEM MOLEKULARGEWICHT
PROCÉDÉ DE PRODUCTION D'ACIDE HYALURONIQUE À FAIBLE POIDS MOLÉCULAIRE

(30) Priority: 15.09.2009 KR 20090087185
(43) Date of publication of application: 25.07.2012
(73) Proprietor: Ildong Pharm Co., Ltd., Seoul 137-733 (KR)
(72) Inventor: KANG, Dae-Jung, Yongin-si Gyeonggi-do 446-582 (KR); IM, Jong-Hyuk, Gwacheon-si Gyeonggi-do 427-739 (KR); KANG, Jae-Hoon, Seoul 135-838 (KR)
(74) Representative: Stolmár & Partner Patentanwälte PartG mbB
(86) International application number: PCT/KR2010/006312
(87) International publication number: WO 2011/034341

(56) References cited:
- CN-A- 101 020 724
- JP-A- 10 195 107
- JP-A- 2002 360 292
- JP-B2- 3 113 056
- KR-A- 20080 046 821
- US-A- 4 278 593
- US-A- 5 756 721
- US-A1- 2009 162 905

## Description

### Technical Field

This application claims priority from and the benefit of Korean Patent Application No. 10-2009-0087185, filed on September 15, 2010.

The present invention relates to a method for lowering a molecular weight of high molecular hyaluronic acid while the high molecular hyaluronic acid's own characteristics such as viscosity, elasticity, and moisture absorption are maintained. More particularly, the present invention relates to a method of producing industrially applicable high-quality low molecular weight hyaluronic acid through decomposition medium to be reacted with hyaluronic acid, and efficient low molecular weight producing condition.

### Background Art

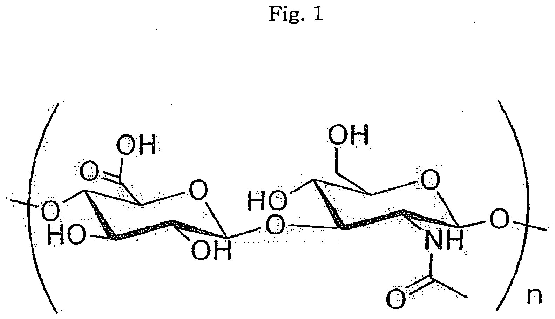

Hyaluronic acid ((HA), Hyaluronan, (C₁₄H₂₀NNaO₁₁) n (n>1000)) is a polymer existing throughout living organisms, and is a polysaccharide, called glycosaminoglycan. It has a structure shown in [FIG. 1], which is composed of alternating D-glucuronic acid and N-acetylglucosamine, linked together via alternating β-1,3 and β-1,4 glycosidic bonds. It is a water-soluble material and its usable molecular weight has a wide range of 1,000 to 13,000,000Da (daltons). Also, it has a structure of a straight chain.

Hyaluronic acid was first found in 1934 by Meyer and Palmer from vitreous humor of a cow's eye, and is distributed in abundance in skin, optical vitreous bodies, joint fluid, muscles, umbilical cords, chicken's comb, etc. Thus, it is obtained from these organs through separation and extraction. Especially, it is known to be present within a placenta or a joint in abundance, and is generally extracted from chicken's comb. However, it is known that in the production of hyaluronic acid from biological tissues, it is difficult to separate a polymer such as chondroitin, and there is a concern about animal-derived pathogenic materials.

The hyaluronic acid with salt structure shows a high efficacy and a high effect, and shows a strong lubricative effect in a physical friction state due to its high moisturizing effect. Also, it has preferable advantages in various effects and properties such as protection against bacterial invasion, etc. Thus, the development of various products using the hyaluronic acid has been recently conducted. These advantages can be applied to medical supplements, bio materials, and foods as well as play a role in medical supplies or cosmetics. Further, novel fields based on hyaluronic acid have been continuously developed.

High molecular weight hyaluronic acid has functional advantages such as increased viscosity, active joint lubrication, and moisture absorption and elasticity. Thus, its use has been widened to various fields of medical supplies, such as ophthalmic/knee joint injection or eye-drops. In consideration of a recent tremendous increase of a hyaluronic acid market all over the world, it is expected that the hyaluronic acid's derivative will be developed.

Unlike the high molecular weight hyaluronic acid, the potency of low molecular weight hyaluronic acid has been underestimated. However, body absorbency of low molecular weight hyaluronic acid has been recently studied. Accordingly, the use of low molecular weight hyaluronic acid in cosmetics or foods requiring tissue absorbency has been spotlighted. Thus, the development of low molecular weight hyaluronic acid is required, and a technology for lowering the molecular weight while maintaining the hyaluronic acid's characteristic function is required.

A method for lowering the molecular weight of high molecular weight hyaluronic acid has been reported in many theses and patents. For example, there is a hydrolysis method by acid and base (Japanese Patent Publication sho 63-57602, Patent Publication Pyung 1-266102, Y. Tokita et al., Polymer Degradation and Stability, 48, 269-273, 1995). This method has a disadvantage in that a new process is added, and after treatment, pH has to be increased or decreased to a value within a standard range. In the method, the change rate of pH may vary according to the concentration of hyaluronic acid. Thus, it is difficult to employ the method in an actual manufacturing process. Also, it was found that the adjustment of pH increases impurities, and especially increases the value of endotoxin. Thus, a purification process for removing impurities has to be added, thereby increasing a cost. In CN 101020724A the hyaluronic acid obtained by fermentation first undergoes a purification process and afterwards the enzyme hyaluronidase is used for degradation of the pure high molecular weight hyaluronic acid to low molecular weight hyaluronic acid. In US 2009/162905 A1 sodium hydroxide is used to reduce the average molecular weight of hyaluronic acid up to a desired value and afterwards a purification procedure is carried out.

Meanwhile, in a decomposition method through ultrasonication or high temperature heating treatment at an appropriate pH, a new machine is required to be introduced, and also in a purification process, an unnecessary cost is caused additionally. Also, in a method using an oxidizing agent such as hypochlorous acid (Japanese Patent Publication Pyung 2-245193), a method using hydrogen peroxide (Japanese Patent Publication Pyung 2-22301), and a method for using a decomposition capability of ammonium persulfate (Korean Registered Patent 10-0369517), hydrolysis can be achieved but an introduced decomposition material has to be removed. Further, the production of endotoxin or other impurities may cause low quality.

In the above mentioned methods for lowering the molecular weight of high molecular weight hyaluronic acid, during a purification process, due to a decomposition promoter newly introduced for adjustment of a molecular weight, only a decomposition action exists. In other words, in a purification process, a quality improvement process of a raw material has to be stopped, and then an additional machine for adjusting the molecular weight has to be introduced, and an additional cost is caused. Thus, the methods are inefficient. In other words, the treatment process becomes more complicated, and the addition of processes prolongs required time, thereby increasing cost. In industrial view of mass production, these are very economically inefficient factors.

### Disclosure

### Technical Problem

Therefore, the inventors of the present invention researched a method for lowering a molecular weight of high molecular weight hyaluronic acid, in which removal of impurities and adjustment of a molecular weight can be achieved at once. Then, they found that when hyaluronic acid is brought into contact with activated carbon under an appropriate condition, the average molecular weight of hyaluronic acid is decreased. Based on this finding, they completed this invention.

The present invention provides a method for lowering the molecular weight of high molecular weight hyaluronic acid. More particularly the present invention provides a method for economically producing high-quality low molecular weight hyaluronic acid, in which inefficiency problems of a conventional method, such as quality degradation caused by the production of impurities, and cost increase caused by the addition of a complicated process are solved.

Accordingly, an object of the present invention is to provide a method for preparing low molecular weight hyaluronic acid comprising the step of bringing hyaluronic acid into contact with activated carbon.

Also, another object of the present invention is to provide a method for lowering the molecular weight of hyaluronic acid comprising the step of bringing hyaluronic acid into contact with activated carbon.

### Technical Solution

To achieve the above-mentioned object, the present invention provides a method for preparing low molecular weight hyaluronic acid comprising the step of bringing hyaluronic acid into contact with activated carbon.

To achieve another object, the present invention provides a method for lowering the molecular weight of hyaluronic acid comprising the step of bringing hyaluronic acid into contact with activated carbon.

The present invention provides a method of using activated carbon as a reaction medium in order to convert high molecular weight hyaluronic acid into low molecular weight hyaluronic acid having a predetermined molecular weight, in which the high molecular weight hyaluronic acid is obtained through microorganism culture, chicken's comb extraction, or the like.

The high molecular weight hyaluronic acid used in the present invention may be obtained through microorganism culture, chicken's comb extraction, or the like. An example of obtaining high molecular weight hyaluronic acid through microorganism culture is as follows.

*Streptococcus* sp. ID9102 (KCTC11395BP) is selected as a hyaluronic acid producing microorganism, and is cultured in a 75 L fermenter with glucose (40-100 g/L), yeast extract (2-5 g/L), casein peptone (10-20 g/L), magnesium sulfate (0.5-1 g/L), potassium dihydrogen phosphate (1-5 g/L), sodium chloride (2-10 g/L), glutamic acid (0.1-1 g/L), at pH 6.0-7.0, at 32-37 °C, under an aerobic condition of 0.1-1 vvm. Then hyaluronic acid having average molecular weights of 2,000,000-4,000,000 Da is produced with productivity in a range of 4-6 g/L.

From a culture containing hyaluronic acid, a microorganism is removed through a known method such as centrifugation, filter press, depth filter, and membrane filtration. Then, the filtrate is used as high molecular weight hyaluronic acid in the present invention.

In the present invention, the high molecular weight hyaluronic acid is decomposed into low molecular weight hyaluronic acid having a specific range of average molecular weight, by activated carbon, and other low molecular weight conversion conditions.

The present invention provides a method for preparing low molecular weight hyaluronic acid comprising the step of bringing hyaluronic acid into contact with activated carbon,
wherein the activated carbon is used as a reaction medium in order to convert high molecular weight hyaluronic acid into low molecular weight hyaluronic acid having a predetermined molecular weight, and
wherein the high molecular weight hyaluronic acid is obtained through microorganism culture or chicken's comb extraction.

The inventive method is characterized in that it comprises the step of bringing hyaluronic acid into contact with activated carbon.

In the present invention, hyaluronic acid ((HA), Hyaluronan, (C₁₄H₂₀NNaO₁₁)ₙ (n>1000)) is a polymer existing throughout living organisms, and is a polysaccharide, called glycosaminoglycan. It has a structure shown in [FIG. 1].

In the present invention, hyaluronic acid may be obtained from a biological tissue such as chicken's comb through purification, and may be produced from a transformed microorganism through a genetic engineering method. The hyaluronic acid used as a raw material in the present invention may be prepared by the above described method or bought from commercial sources. The average molecular weight of hyaluronic acid used in the method of the present invention is not particularly limited. For example, the average molecular weight of hyaluronic acid used in the present invention may range from 100,000 Da to 13,000,000 Da, from 1,000,000 Da to 13,000,000 Da, from 3,500,000 Da to 13,000,000 Da, from 3,500,000 Da to 10,000,000 Da, from 3,500,000 Da to 8,000,000 Da, preferably range from 3,500,000 Da to 13,000,000 Da, and more preferably range from 3,500,000 Da to 8,000,000 Da.

In one embodiment of the present invention, *Streptococcus sp.* ID9102 (KCTC11395BP) capable of producing hyaluronic acid was selected as a hyaluronic acid producing microorganism, and was cultured in a 75 L fermenter with glucose (40-100 g/L), yeast extract (2-5 g/L), casein peptone (10-20 g/L), magnesium sulfate (0.5-1 g/L), potassium dihydrogen phosphate (1-5 g/L), sodium chloride (2-10 g/L), glutamic acid (0.1-1 g/L), at pH 6.0-7.0, at 32-37 °C, under an aerobic condition of 0.1-1 vvm so as to obtain hyaluronic acid.

Activated carbon indicates a carbonaceous material having high adsorptivity. It is obtained by treating wood, lignite, or peat with an activator such as zinc chloride or phosphoric acid, followed by drying, or is obtained by activating charcoal with water vapor. In general, activated carbon is made in a powder state or in a particle state. Powder-type activated carbon may be made into particle-type activated carbon for use. It is mainly used as an adsorbent for absorbing gases or moisture, and may be used for various purposes such as solvent recovery, gas purification, decoloring, or the like.

In the present invention, activated carbon may be prepared directly, or bought from commercial sources. There is no specific limitation in the kind of the activated carbon used in the present invention. For example, it may be granular activated carbon, powder activated carbon, or palletized activated carbon, and preferably may be powder activated carbon. Powder activated carbon may be obtained by a steam activating method or a chemical activating method. There is no specific limitation in the activating method.

In the step of bringing hyaluronic acid into contact with activated carbon, there is no specific limitation in the contact method. Preferably, a hyaluronic acid-containing purified culture medium prepared by a microorganism culture method, or a hyaluronic acid diluent solution containing hyaluronic acid diluted in a solvent may be dispersed in activated carbon. The solvent for diluting the hyaluronic acid is not particularly limited. For example, it may be water or a mixed solvent of lower alcohol having 1 to 6 carbon atoms and water. Preferably, it may be water.

In the hyaluronic acid-containing purified culture medium or in the hyaluronic acid diluent solution, the concentration of hyaluronic acid is not particularly limited, and may range from 0.01 to 90%, from 0.1 to 50%, from 0.1 to 30%, or from 0.1 to 10%. Preferably, it may range from 0.1 to 10%.

Through the contact, a reaction of the hyaluronic acid is caused. As a result, the molecular weight is reduced. The reaction indicates a phenomenon in a response to stimulation, or a chemical change occurring between materials.

The method of the present invention may have further steps for harvesting hyaluronic acid from the reaction solution after the reaction was terminated. Any method is available for harvesting hyaluronic acid from the reaction solution as long as it is well known for isolation and purification method. Generally, centrifugation, filtration and the like may be performed for removing activated carbon and electrophoresis and various column chromatographies may also be performed. For the chromatographies, ion exchange chromatography, gel-filtration chromatography, HPLC, reversed-phase chromatography, affinity column chromatography alone or in combination thereof may be used for harvesting hyaluronic acid.

The method of the present invention has an effect on lowing molecular weight of hyaluronic acid.

In the present invention, the ratio of hyaluronic acid and activated carbon is in a range from 1: 2 to 1: 6.

In the present invention, there is no specific limitation in the temperature and time required for bringing hyaluronic acid into contact with activated carbon. They may vary according to the molecular weight of hyaluronic acid before contact with activated carbon, and the required molecular weight of hyaluronic acid. Preferably, the temperature may range from 25 °C to 45 °C, and more preferably the temperature may range from 35 °C to 45 °C. The time may preferably range from 3 to 18 hours, and more preferably range from 6 to 18 hours.

As the ratio of activated carbon or the contact temperature increases, a reduction ratio of the molecular weight of hyaluronic acid per unit time increases. As the contact time increases, the average molecular weight of hyaluronic acid obtained after the contact decreases. The average molecular weight of hyaluronic acid prepared by the inventive method may vary according to the molecular weight of hyaluronic acid before contact with activated carbon, the ratio of activated carbon, the contact temperature, and the contact time. Thus, it is not particularly limited. For example, the average molecular weight of hyaluronic acid produced in the present invention may range from 10,000 Da to 3,000,000 Da, from 10,000 Da to 100,000 Da, from 100,000 Da to 500,000 Da, from 500,000 Da to 1,000,000 Da, from 1,000,000 Da to 2,000,000 Da, from 2,000,000 Da to 3,000,000 Da, from 3,000,000 Da to 4,000,000 Da, from 4,000,000 Da to 5,000,000 Da, from 5,000,000 Da to 6,000,000 Da, from 6,000,000 Da to 7,000,000 Da, from 7,000,000 Da to 8,000,000 Da, from 8,000,000 Da to 9,000,000 Da. Preferably, it may range from 10,000 Da to 3,000,000 Da, and more preferably may range from 10,000 Da to 2,000,000 Da.

The method of the present invention has a simple process, and has easily controllable factors (such as amount of activated carbon, temperature and time) for controlling a reduction ratio of a hyaluronic acid molecular weight. According to a change of control factors, the reduction ratio of a molecular weight of hyaluronic acid is relatively regularly changed. This is very useful in preparation of hyaluronic acid having a predetermined molecular weight. Also, such a characteristic can remove a large amount of endotoxin contained in a culture medium in a case where the hyaluronic acid is prepared by a microorganism culture.

According to the preparation method of the present invention, it is possible to efficiently produce hyaluronic acid having a specific molecular weight by a simple process. Also, it is possible to easily and stably adjust the molecular weight of produced hyaluronic acid through adjustment of the amount of activated carbon, the temperature and the time.

Meanwhile, the present invention provides a method for lowering the molecular weight of hyaluronic acid comprising the step of bringing hyaluronic acid into contact with activated carbon, wherein the activated carbon is used as a reaction medium in order to convert high molecular weight hyaluronic acid into low molecular weight hyaluronic acid having a predetermined molecular weight, and wherein the high molecular weight hyaluronic acid is obtained through microorganism culture or chicken's comb extraction.

It is disclosed for the first time in the present invention to lowering the molecular weight of hyaluronic acid by bringing hyaluronic acid into contact with activated carbon.

In the method of the present invention, hyaluronic acid to be brought into contact with activated carbon, the hyaluronic acid's molecular weight, the activated carbon, and the kind of the activated carbon, a ratio of hyaluronic acid to activated carbon, a contact method, a contact condition (solvent, hyaluronic acid's concentration, and the like), a contact temperature, a contact time, a contact effect, a lowered molecular weight of hyaluronic acid obtained after being brought into contact with activated carbon, and other effects are the same as those described in the above preparation method.

These characteristics of the present invention can be easily understood with reference to Examples of the present invention.

According to an Example of the present invention, a *Streptococcus* sp. microorganism producing hyaluronic acid was cultured so as to produce hyaluronic acid. Then, the hyaluronic acid was purified. Activated carbon with various ratios was dispersed in the purified culture medium and was brought into contact with hyaluronic acid under various temperature conditions with various times. Then, the hyaluronic acid was collected, and its molecular weight was measured.

Under a condition of 25 °C to 45 °C, when the hyaluronic acid was brought into contact with activated carbon for 3 to 18 hours, 3,500,000 Da hyaluronic acid was prepared into hyaluronic acid having various molecular weights of 10,000 Da to 3,000,000 Da.

According to another Example of the present invention, hyaluronic acid extracted and purified from chicken's comb was used in the same experiment through dilution. Then, the result was measured.

As a result, when the hyaluronic acid extracted/purified from chicken's comb was used, it was possible to prepare hyaluronic acid having a lowered molecular weight in the same manner as that in the culture of a microorganism producing hyaluronic acid.

Thus, through the method of the present invention, it is possible to efficiently reduce the molecular weight of hyaluronic acid irrespective of a hyaluronic acid preparation method or a contact condition between hyaluronic acid and activated carbon.

In one Example of the present invention, 1 t of hyaluronic acid having an average molecular weight of 3,500,000 Da was cultured, and was reacted with activated carbon in an amount of 4 times the weight of hyaluronic acid for 4 hours at a reaction temperature of 45 °C. Then, hyaluronic acid with an average molecular weight of 2,000,000 Da was prepared.

In other Examples of the present invention, under the same condition as that in Example above, for various reaction times of 6, 7 or 8 hours, hyaluronic acid was prepared, and the average molecular weight was measured. As a result, according to the reaction time, hyaluronic acid with average molecular weights of 1,000,000 Da, 500,000 Da and 100,000 Da was prepared.

Accordingly, in the inventive method, it is possible to easily adjust the molecular weight of hyaluronic acid by adjusting various factors such as the ratio of activated carbon, the temperature, the reaction time. Further, the inventive method is an industrially applicable high-quality low molecular weight hyaluronic acid preparation method.

### Advantageous Effects

The method of the present invention for lowering a molecular weight of high molecular hyaluronic acid has a convenient process without the inconvenience of reprocessing for the removal of input materials, or requiring pH treatment, various reaction catalysts, and complicated additional treatment conditions (such as heat treatment) as in a conventional method. Furthermore, the method has an effect for removing impurities. Thus, through the method, it is possible to conveniently and economically produce low molecular weight hyaluronic acid with high purity. The inventive method for lowering the molecular weight has an advantage in that according to a change of a reaction condition using activated carbon, the molecular weight of low molecular weight hyaluronic acid can be variously adjusted. The low molecular weight hyaluronic acid produced by the method of the present invention can be produced in accordance with the standards of medical supplies while the hyaluronic acid's own characteristic can be maintained. Also, it can be produced in accordance with the standards of cosmetics or foods.

### Description of Drawings

FIG. 1 shows a repeating unit of hyaluronic acid, which is composed of alternating D-glucuronic acid and N-acetylglucosamine, linked together via alternating β-1,3 and β-1,4;

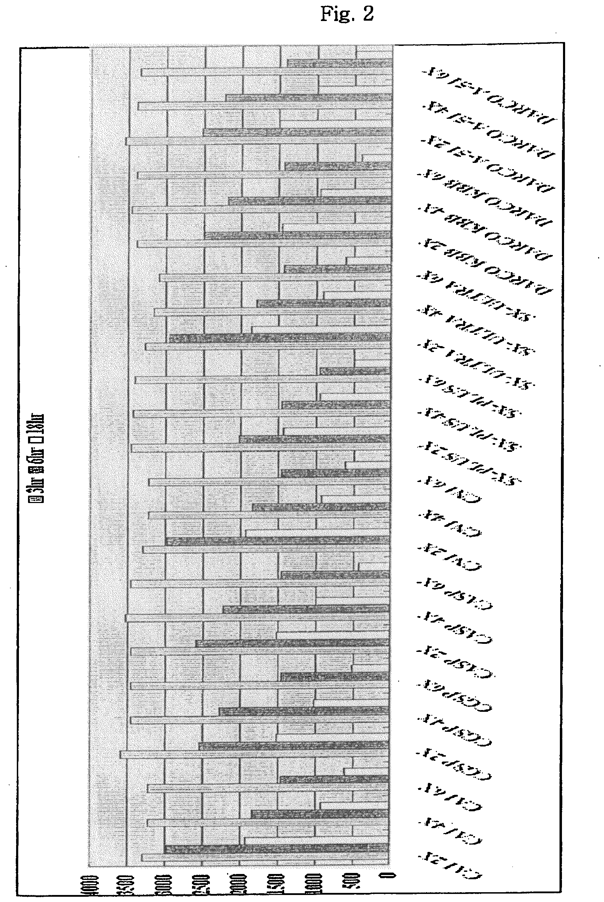

FIG. 2 is a graph showing the change of molecular weight of hyaluronic acid according to various kinds of activated carbon and respective concentrations at 25 °C (2X, 4X, 6X : amount of administered activated carbon with respect to hyaluronic acid, CA1, CGSP, CASP, CN1, SX-PLUS, SX-ULTRA, DARCO KBB, DARCO A-51: the kind of used activated carbon, Norit® CA1, Norit® CGSP, Norit® CASP, Norit® CN1, Norit® SX-PLUS, Norit® SX-ULTRA, Norit@ DARCO KBB, Norit® DARCO A-51);

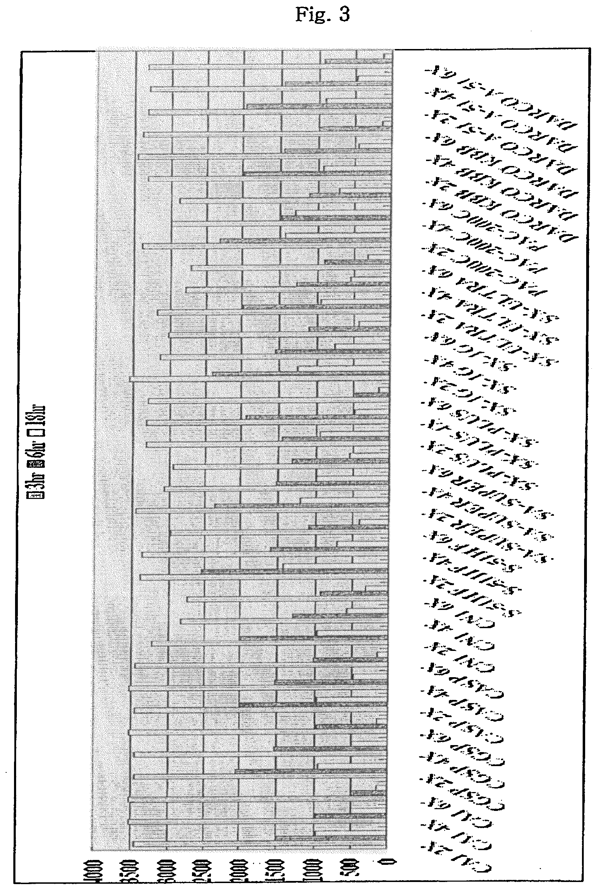

FIG. 3 is a graph showing the change of molecular weight of hyaluronic acid according to various kinds of activated carbon, and respective concentrations at 35 °C(2X, 4X, 6X : amount of administered activated carbon with respect to hyaluronic acid, CA1, CGSP, CASP, CN1, S-51HF, SA-SUPER, SX-PLUS, SX-1G, SX-ULTRA, PAC-200C, DARCO KBB, DARCO A-51: the kind of used activated carbon, Norit® CA1, Norit® CGSP, Norit® CASP, Norit@ CN1, Norit® S-51HF, Norit® SA-SUPER, Norit® SX-PLUS, Norit® SX-1G, Norit® SX-ULTRA, Norit® PAC-200C, Norit® DARCO KBB, Norit® DARCO A-51) and

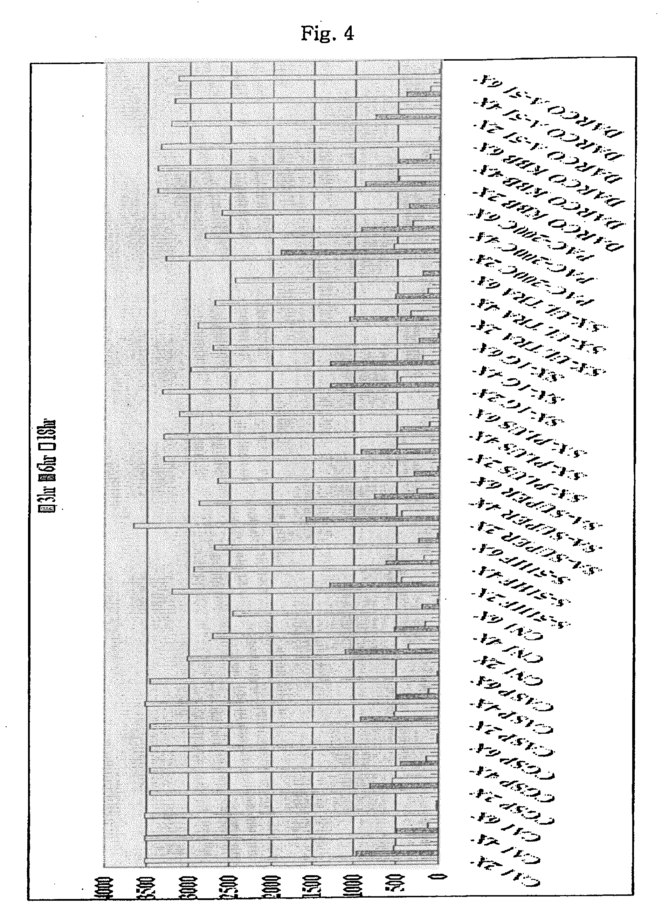

FIG. 4 is a graph showing the change of molecular weight of hyaluronic acid according to various kinds of activated carbon, and respective concentrations at 45 °C(2X, 4X, 6X : amount of administered activated carbon with respect to hyaluronic acid, CA1, CGSP, CASP, CN1, S-51HF, SA-SUPER, SX-PLUS, SX-1G, SX-ULTRA, PAC-200C, DARCO KBB, DARCO A-51: the kind of used activated carbon, Norit@ CA1, Norit® CGSP, Norit® CASP, Norit® CN1, Norit® S-51HF, Norit® SA-SUPER, Norit® SX-PLUS, Norit® SX-1G, Norit® SX-ULTRA, Norit® PAC-200C, Norit® DARCO KBB, Norit® DARCO A-51).

### Mode for Invention

Hereinafter, exemplary embodiments of the present invention will be described with reference to the accompanying drawings.

In the present invention, a hyaluronic acid concentration existing in a solution was found by a carbazole method (T. Bitter, Anal. Biochem., 1962, 4, 330-334). The average molecular weight of hyaluronic acid was obtained by gel chromatography (Narlin B. Beaty et al, Anal. Biochem., 1985, 147, 387-395). Analysis conditions include a column of Toyo Soda TSK gel G6000PWXL, and a moving phase of 150 mM NaCl, 3 mM Na2HPO4 (pH 7.0), 0.02% NaN₃. For detection, a refractive index detector (Shodex) was used. As a reference material, polyethylene oxide was prepared at 2 mg/mℓ concentration. As an endotoxin, a LAL reagent commercially available from Charles River Laboratories Korea was quantified. Within a range less than a maximum effective dilution multiple, the dilution multiple was set as 3 point. It was determined that from a negative control group, endotoxin was not detected, and through a positive product control, there was no reaction interference factor.

In Examples below, a concentration of each of high molecular weight hyaluronic acid extracted from a microorganism, and high molecular weight hyaluronic acid extracted from chicken's comb is adjusted to a predetermined value, and then low molecular weight hyaluronic acid is produced according to a change of a reaction condition by using activated carbon as a medium for producing low molecular weight hyaluronic acid. The change of the average molecular weight of produced low molecular weight hyaluronic acid will be described later.

The following examples illustrate the invention and are not intended to limit the same.

### <Example 1>

### Method for producing low molecular weight hyaluronic acid by using CA1 activated carbon

In this Example, as a raw material for producing low molecular weight hyaluronic acid, a composition purified from a culture obtained through culturing of *Streptococcus* sp. ID9102 (KCTC11395BP) as a producing microorganism was used. The average molecular weight of hyaluronic acid included in the raw material is 3,500,000 Da, and the concentration of the endotoxin is much greater than 0.5 EU/mg.

The culturing conditions are as follows:
*Streptococcus* sp. ID9102 (KCTC11395BP) was selected as a microorganism producing hyaluronic acid, and is cultured in a 75 L fermenter containing glucose (40-100 g/L), yeast extract (2-5 g/L), casein peptone (10-20 g/L), magnesium sulfate (0.5-1 g/L), potassium dihydrogen phosphate (1-5 g/L), sodium chloride (2-10 g/L), and glutamic acid (0.1-1 g/L), at pH 6.0-7.0, at 32-37 °C, under an aerobic condition of 0.1-1 vvm.

The basic reaction conditions are as follows:
In order to obtain an appropriate agitating force and reactivity of hyaluronic acid purified from ultrafiltration and Norit® CA1 (hereinafter referred to as CA1, Norit, netherlands, Norit® - activated carbons used in Examples were produced from Norit) activated carbon, the concentration of hyaluronic acid was adjusted to 2.5g HA/L. Then, the hyaluronic acid was introduced in an amount of 300 mℓ into a 1 L glass beaker, and CA1 activated carbon was introduced into a glass beaker at a concentration twice, 4 times and 6 times greater than hyaluronic acid concentration. Then, impeller (diameter 5 cm) made of teflon was rotated at a 300 rpm rate while the predetermined reaction condition was maintained. In order to prevent mixing of impurities or evaporation of a reaction liquid to the maximum until the completion of the reaction, impeller portions other than the rotation portion of the impeller were sealed with a cover. Reaction temperatures were 25 °C, 35 °C, and 45 °C.

After 3, 6, and 18 hours, each sample was collected, and an endotoxin, a hyaluronic acid concentration, and an average molecular weight were analyzed according to the analysis method. The result is noted in [table 1]. According to an increase of a reaction temperature, the decomposition capability of CA1 activated carbon increased, thereby increasing the lowering rate of the molecular weight of hyaluronic acid. At 25 °C, under a condition of CA1 2X, for 6 hours, 2,000,000 Da of hyaluronic acid was obtained, and for 18 hours, 1,500,000 Da of molecular weight was obtained. At 25 °C, and CA1 4X, for 18 hours, about 1,000,000 Da of molecular weight was obtained, and at CA1 6X, for 6 hours, about 1,000,000 Da of molecular weight was obtained, and for 18 hours, 500,000 Da of molecular weight was obtained. In a case where the reaction was carried out at 35 °C, although the concentration had a reactivity 2X less than that at 25 °C, the molecular weight decomposition capability was similar to that at 25 °C. At 35 °C, with 6X, for 18 hours, 130,000 Da of molecular weight was obtained. In a case where the reaction was carried out at 45 °C, although the concentration had a reactivity 2X less than that at 35 °C, the molecular weight decomposition capability was similar to that at 35 °C. With 6X, 15,000 Da of molecular weight was obtained. Under all reaction conditions, the endotoxin was less than 0.5 EU/mg. Also, it was found that an increase of the reaction temperature is effective in further reduction of the endotoxin. Also, the concentration of hyaluronic acid was maintained at 92% or more, under all reaction conditions. Thus, it was found that there is no concern about the loss of hyaluronic acid.

**Table 1**

| Result after reaction of hyaluronic acid with CA1 activated carbon | | | | | | | | | | |
|---|---|---|---|---|---|---|---|---|---|---|
| Rxn Temp. | Conc. Of Acticated carbon | MW, kDa | | | Endotoxin, EU/mg | | | g HA/L | | |
| | | 3h | 6h | 18h | 3h | 6h | 18h | 3h | 6h | 18h |
| 25°C | 2X | 3519 | 2089 | 1485 | <0.5 | <0.5 | <0.5 | 2.51 | 2.48 | 2.34 |
| 25°C | 4X | 3519 | 1485 | 994 | <0.5 | <0.5 | <0.5 | 2.52 | 2.46 | 2.44 |
| 25°C | 6X | 3519 | 975 | 523 | <0.5 | <0.5 | <0.5 | 2.50 | 2.43 | 2.41 |
| 35°C | 2X | 3449 | 1485 | 975 | <0.05 | <0.05 | <0.05 | 2.50 | 2.47 | 2.31 |
| 35°C | 4X | 3519 | 975 | 513 | <0.05 | <0.05 | <0.05 | 2.50 | 2.45 | 2.42 |
| 35°C | 6X | 3519 | 503 | 134 | <0.05 | <0.05 | <0.05 | 2.47 | 2.41 | 2.39 |
| 45°C | 2X | 3519 | 955 | 523 | <0.005 | <0.005 | <0.005 | 2.49 | 2.45 | 2.32 |
| 45°C | 4X | 3519 | 473 | 129 | <0.005 | <0.005 | <0.005 | 2.51 | 2.44 | 2.42 |
| 45°C | 6X | 3519 | 16 | 15 | <0.005 | <0.005 | <0.005 | 2.47 | 2.41 | 2.38 |

### <Example 2>

### Method of preparing low molecular weight hyaluronic acid using CGSP activated carbon

The Hyaluronic acid and the reaction condition of <Example 1> was used except Norit® CGSP (Hereafter CGSP)instead of CA1 activated carbon as a medium for producing low molecular weight hyaluronic acid to perform comparative analysis of productivity of low molecular hyaluronic acid.

After 3, 6, and 18 hours, each sample was collected, and an endotoxin, a hyaluronic acid concentration, and an average molecular weight were analyzed according to the analysis method. The result is noted in [table 2]. Like CA1 activated carbon, according to an increase of a reaction temperature, the decomposition capability of CGSP activated carbon increased, thereby increasing the lowering rate of the molecular weight of hyaluronic acid. At 25 °C, under a condition of CGSP 4X, for 18 hours, 1,000,000 Da of hyaluronic acid was obtained, and 6x for 18 hours, 500,000 Da of molecular weight was obtained. In a case where the reaction was carried out at 35 °C, although the concentration had a reactivity 2X less than that at 25 °C, the molecular weight decomposition capability was similar to that at 25 °C. Especially, in case of 6x at 45 °C, the result reached to 10,000 Da of molecular weight. At this reaction conditions, the inventors confirmed that the endotoxin was less than 0.5 EU/mg. Like the result of CA1, it was found that an increase of the reaction temperature is effective in further reduction of the endotoxin. Also, the concentration of hyaluronic acid was maintained at least 93% or more under all reaction conditions. Thus, the inventors confirmed that stability of hyaluronic acid and effect of CGSP activated carbon.

**Table 2**

| Result after reaction of hyaluronic acid with CGSP activated carbon | | | | | | | | | | |
|---|---|---|---|---|---|---|---|---|---|---|
| Rxn Temp. | Conc. Of Acticated carbon | MW, kDa | | | Endotoxin, EU/mg | | | g HA/L | | |
| | | 3h | 6h | 18h | 3h | 6h | 18h | 3h | 6h | 18h |
| 25°C | 2X | 3590 | 2553 | 1515 | <0.5 | <0.5 | <0.5 | 2.52 | 2.59 | 2.47 |
| 25°C | 4X | 3449 | 2263 | 1015 | <0.5 | <0.5 | <0.5 | 2.52 | 2.49 | 2.50 |
| 25°C | 6X | 3449 | 1456 | 513 | <0.5 | <0.5 | <0.5 | 2.48 | 2.45 | 2.40 |
| 35°C | 2X | 3449 | 2047 | 936 | <0.05 | <0.05 | <0.05 | 2.52 | 2.45 | 2.39 |
| 35°C | 4X | 3449 | 1515 | 503 | <0.05 | <0.05 | <0.05 | 2.51 | 2.50 | 2.41 |
| 35°C | 6X | 3519 | 975 | 134 | <0.05 | <0.05 | <0.05 | 2.50 | 2.48 | 2.41 |
| 45°C | 2X | 3449 | 814 | 534 | <0.005 | <0.005 | <0.005 | 2.48 | 2.42 | 2.39 |
| 45°C | 4X | 3449 | 437 | 139 | <0.005 | <0.005 | <0.005 | 2.47 | 2.45 | 2.34 |
| 45°C | 6X | 3449 | 18 | 12 | <0.005 | <0.005 | <0.005 | 2.45 | 2.40 | 2.32 |

### <Example 3>

### Method of preparing low molecular weight hyaluronic acid using CASP activated carbon

The reaction condition of <Example 1> was used except Norit® CASP (Hereafter CASP) instead of CA1 activated carbon to perform comparative analysis of productivity of low molecular hyaluronic acid.

After 3, 6, and 18 hours, each sample was collected, and an endotoxin, a hyaluronic acid concentration, and an average molecular weight were analyzed according to the analysis method. The result is noted in [table 3]. Like CA1 and CGSP activated carbon, according to an increase of a reaction temperature, the decomposition capability of CASP activated carbon also increased, thereby increasing the lowering rate of the molecular weight of hyaluronic acid. At 25 °C, under a condition of CASP 4X, for 18 hours, 1,000,000 Da of hyaluronic acid was obtained. In a case where the reaction was carried out at 35 °C, although the concentration had a reactivity 2X less than that at 25 °C, the molecular weight decomposition capability was similar to that at 25 °C. In case of at 45 °C, the decomposition capability was highly increased and in case of 2x for 6 hrs, 1,000,000 Da, for 18 hrs, 5000,000 Da of hyaluronic acid was obtained and in case of 2S, for 6 hrs, 1,000,000 Da, for 18 hrs, 500,000 Da, 18 hrs, 100,000 Da of hyaluronic acid was obtained. In case of 6x, the decomposition capability was increased most highly, therefore the present inventors confirmed that for 6 hrs, 15,000 Da and for 18 hrs, 12,000 Da of hyaluronic acid was obtained. The inventors confirmed that the endotoxin was less than 0.5 EU/mg and it was found that an increase of the reaction temperature is effective in further reduction of the endotoxin like the result of CA1 and CGSP. Also, the concentration of hyaluronic acid was maintained at least 93% or more under all reaction conditions of CASP.

**Table 3**

| Result after reaction of hyaluronic acid with CASP activated carbon | | | | | | | | | | |
|---|---|---|---|---|---|---|---|---|---|---|
| Rxn Temp. | Conc. Of Acticated carbon | MW, kDa | | | Endotoxin, EU/mg | | | g HA/L | | |
| | | 3h | 6h | 18h | 3h | 6h | 18h | 3h | 6h | 18h |
| 25°C | 2X | 3449 | 2605 | 1515 | <0.5 | <0.5 | <0.5 | 2.53 | 2.50 | 2.35 |
| 25°C | 4X | 3519 | 2218 | 994 | <0.5 | <0.5 | <0.5 | 2.51 | 2.48 | 2.43 |
| 25°C | 6X | 3449 | 1456 | 428 | <0.5 | <0.5 | <0.5 | 2.55 | 2.42 | 2.42 |
| 35°C | 2X | 3449 | 2007 | 955 | <0.05 | <0.05 | <0.05 | 2.52 | 2.47 | 2.33 |
| 35°C | 4X | 3519 | 1515 | 483 | <0.05 | <0.05 | <0.05 | 2.48 | 2.46 | 2.42 |
| 35°C | 6X | 3449 | 1015 | 129 | <0.05 | <0.05 | <0.05 | 2.54 | 2.40 | 2.41 |
| 45°C | 2X | 3449 | 918 | 513 | <0.005 | <0.005 | <0.005 | 2.50 | 2.47 | 2.32 |
| 45°C | 4X | 3519 | 483 | 126 | <0.005 | <0.005 | <0.005 | 2.50 | 2.44 | 2.42 |
| 45°C | 6X | 3449 | 16 | 10 | <0.005 | <0.005 | <0.005 | 2.49 | 2.42 | 2.38 |

### <Example 4>

### Method of preparing low molecular weight hyaluronic acid using CN1 activated carbon

The reaction condition of <Example 1> was used except Norit® CN1 (Hereafter CN1)instead of CA1 activated carbon to perform comparative analysis of productivity of low molecular hyaluronic acid.

After 3, 6, and 18 hours, each sample was collected, and an endotoxin, a hyaluronic acid concentration, and an average molecular weight were analyzed according to the analysis method. The result is noted in [table 4]. According to an increase of a reaction temperature, the decomposition capability of CASP activated carbon also increased, thereby increasing the lowering rate of the molecular weight of hyaluronic acid. At 25 °C, under a condition of CN1 4X, for 18 hours, 1,000,000 Da of hyaluronic acid was obtained. In a case where the reaction was carried out at 35 °C, the decomposition capability was highly increased and in case of 4x for 6 hrs, 1,000,000 Da, for 18 hrs, 5000,000 Da of hyaluronic acid was obtained. In case of at 45 °C, 2x for 6 hrs, 1,000,000 Da, 4x for 6 hrs 500,000 Da of hyaluronic acid was obtained. The inventors confirmed that the endotoxin was less than 0.5 EU/mg and it was found that an increase of the reaction temperature is effective in further reduction of the endotoxin same as the result of the above results. Also, the concentration of hyaluronic acid was maintained at least 93% or more under all reaction conditions of CN1.

**Table 4**

| Result after reaction of hyaluronic acid with CN1 activated carbon | | | | | | | | | | |
|---|---|---|---|---|---|---|---|---|---|---|
| Rxn Temp. | Conc. Of Acticated carbon | *MW, kDa* | | | Endotoxin, EU/mg | | | g HA/L | | |
| | | *3h* | *6h* | *18h* | 3h | 6h | 18h | 3h | 6h | 18h |
| 25°C | 2X | 3302 | 2981 | 1932 | <0.5 | <0.5 | <0.5 | 2.51 | 2.51 | 2.48 |
| 25°C | 4X | 3236 | 1832 | 937 | <0.5 | <0.5 | <0.5 | 2.5 | 2.48 | 2.44 |
| 25°C | 6X | 3221 | 1456 | 612 | <0.5 | <0.5 | <0.5 | 2.52 | 2.49 | 2.43 |
| 35°C | 2X | 3209 | 2007 | 955 | <0.05 | <0.05 | <0.05 | 2.52 | 2.47 | 2.45 |
| 35°C | 4X | 2836 | 1284 | 583 | <0.05 | <0.05 | <0.05 | 2.48 | 2.46 | 2.42 |
| 35°C | 6X | 2732 | 916 | 329 | <0.05 | <0.05 | <0.05 | 2.54 | 2.5 | 2.49 |
| 45°C | 2X | 3018 | 1096 | 363 | <0.005 | <0.005 | <0.005 | 2.5 | 2.47 | 2.35 |
| 45°C | 4X | 2701 | 530 | 151 | <0.005 | <0.005 | <0.005 | 2.49 | 2.46 | 2.45 |
| 45°C | 6X | 2451 | 206 | 11 | <0.005 | <0.005 | <0.005 | 2.48 | 2.47 | 2.39 |

### <Example 5>

### Method of preparing low molecular weight hyaluronic acid using S-51HF activated carbon

The reaction condition of <Example 1> was used except Norit® S-51HF (Hereafter S-51HF)instead of CA1 activated carbon to perform comparative analysis of productivity of low molecular hyaluronic acid.

After 3, 6, and 18 hours, each sample was collected, and an endotoxin, a hyaluronic acid concentration, and an average molecular weight were analyzed according to the analysis method. The result is noted in [table 5]. According to an increase of a reaction temperature, the decomposition capability of S-51HF activated carbon also increased, thereby increasing the lowering rate of the molecular weight of hyaluronic acid. At 35 °C, under a condition of S-51HF 6X, for 6 hours, 1,000,000 Da of hyaluronic acid was obtained. In a case where the reaction was carried out at 45 °C, 2x for 6 hrs, 1,000,000 Da, 4x for 6 hrs, 5000,000 Da of hyaluronic acid was obtained and in case of 6x for 18 hrs, 1,000,000 Da of hyaluronic acid was obtained. The inventors confirmed that the endotoxin was less than 0.5 EU/mg and it was found that an increase of the reaction temperature is effective in further reduction of the endotoxin same as the result of the above results. Also, the concentration of hyaluronic acid was maintained at least 93% or more under all reaction conditions of S-51HF.

**Table 5**

| Result after reaction of hyaluronic acid with 51HF activated carbon | | | | | | | | | | |
|---|---|---|---|---|---|---|---|---|---|---|
| Rxn Temp. | Conc. Of Acticated carbon | *MW, kDa* | | | Endotoxin, EU/mg | | | gHA/L | | |
| | | *3h* | *3h* | *18h* | 3h | 6h | 18h | 3h | 6h | 18h |
| 35°C | 2X | 3382 | 2558 | 1416 | <0.05 | <0.05 | <0.05 | 2.51 | 2.46 | 2.44 |
| 35°C | 4X | 3367 | 1592 | 724 | <0.05 | <0.05 | <0.05 | 2.47 | 2.45 | 2.41 |
| 35°C | 6X | 2974 | 1077 | 418 | <0.05 | <0.05 | <0.05 | 2.53 | 2.49 | 2.48 |
| 45°C | 2X | 3186 | 1288 | 452 | <0.005 | <0.005 | <0.005 | 2.49 | 2.46 | 2.34 |
| 45°C | 4X | 2941 | 623 | 188 | <0.005 | <0.005 | <0.005 | 2.48 | 2.45 | 2.44 |
| 45°C | 6X | 2669 | 242 | 13 | <0.005 | <0.005 | <0.005 | 2.47 | 2.46 | 2.38 |

### <Example 6>

### Method of preparing low molecular weight hyaluronic acid using SA-SUPER activated carbon

The reaction condition of <Example 1> was used except Norit® SA-SUPER (Hereafter SA-SUPER)instead of CA1 activated carbon to perform comparative analysis of productivity of low molecular hyaluronic acid.

After 3, 6, and 18 hours, each sample was collected, and an endotoxin, a hyaluronic acid concentration, and an average molecular weight were analyzed according to the analysis method. The result is noted in [table 6]. At 35 °C, under a condition of SA-SUPER 2X and 4X, for 18 hours, 500,000 Da, in condition of 6X for 18 hrs, 1000,000 Da of hyaluronic acid was obtained. The inventors confirmed that the endotoxin was less than 0.5 EU/mg and it was found that an increase of the reaction temperature is effective in further reduction of the endotoxin same as the result of the above results. Also, the concentration of hyaluronic acid was maintained at least 93% or more under all reaction conditions of SA-SUPER.

**Table 6**

| Result after reaction of hyaluronic acid with SA-SUPER activated carbon | | | | | | | | | | |
|---|---|---|---|---|---|---|---|---|---|---|
| Rxn Temp. | Conc. Of Acticated carbon | *MW, kDa* | | | Endotoxin, EU/mg | | | g HA/L | | |
| | | *3h* | *6h* | *18h* | 3h | 6h | 18h | 3h | 6h | 18h |
| 35°C | 2X | 3458 | 2359 | 1191 | <0.05 | <0.05 | <0.05 | 2.56 | 2.49 | 2.47 |
| 35°C | 4X | 3049 | 1509 | 984 | <0.05 | <0.05 | <0.05 | 2.52 | 2.48 | 2.35 |
| 35°C | 6X | 2944 | 1318 | 555 | <0.05 | <0.05 | <0.05 | 2.58 | 2.52 | 2.51 |
| 45°C | 2X | 3650 | 1578 | 452 | <0.005 | <0.005 | <0.005 | 2.47 | 2.45 | 2.32 |
| 45°C | 4X | 2852 | 763 | 254 | <0.005 | <0.005 | <0.005 | 2.53 | 2.48 | 2.36 |
| 45°C | 6X | 2642 | 296 | 13 | <0.005 | <0.005 | <0.005 | 2.5 | 2.43 | 2.35 |

### <Example 7>

### Method of preparing low molecular weight hyaluronic acid using SX-PLUS activated carbon

The reaction condition of <Example 1> was used except Norit® SX-PLUS (Hereafter SX- PLUS)instead of CA1 activated carbon to perform comparative analysis of productivity of low molecular hyaluronic acid.

After 3, 6, and 18 hours, each sample was collected, and an endotoxin, a hyaluronic acid concentration, and an average molecular weight were analyzed according to the analysis method. The result is noted in [table 7]. At 25 °C, under a condition of SX-SUPER 6X, for 18 hours, 1,000,000 Da of hyaluronic acid was obtained. At 35 °C 2X for 18 hrs, 1,000,000 Da of hyaluronic acid was obtained and 4x for 18 hrs, 500,000 Da of hyaluronic acid was obtained. At 45 °C 2X for 6 hrs, 1,000,000 Da, 4x for 6 hrs, 500,000 Da of hyaluronic acid was obtained and 6x for 18 hrs, 10,000 Da of hyaluronic acid was obtained. The inventors confirmed that the endotoxin was less than 0.5 EU/mg and it was found that an increase of the reaction temperature is effective in further reduction of the endotoxin same as the result of the above results. Also, the concentration of hyaluronic acid was maintained at least 93% or more under all reaction conditions of SX-PLUS.

**Table 7**

| Result after reaction of hyaluronic acid with SX-PLUS activated carbon | | | | | | | | | | |
|---|---|---|---|---|---|---|---|---|---|---|
| Rxn Temp. | Conc. Of Acticated carbon | *MW, kDa* | | | Endotoxin, EU/mg | | | g HA/L | | |
| | | *3h* | *6h* | *18h* | 3h | 6h | 18h | 3h | 6h | 18h |
| 25°C | 2X | 3268 | 2951 | 1854 | <0.5 | <0.5 | <0.5 | 2.48 | 2.41 | 2.39 |
| 25°C | 4X | 3171 | 1795 | 899 | <0.5 | <0.5 | <0.5 | 2.50 | 2.47 | 2.33 |
| 25°C | 6X | 3092 | 1426 | 599 | <0.5 | <0.5 | <0.5 | 2.49 | 2.46 | 2.41 |
| 35°C | 2X | 3176 | 1966 | 945 | <0.05 | <0.05 | <0.05 | 2.54 | 2.51 | 2.46 |
| 35°C | 4X | 2779 | 1271 | 559 | <0.05 | <0.05 | <0.05 | 2.48 | 2.41 | 2.39 |
| 35°C | 6X | 2704 | 897 | 322 | <0.05 | <0.05 | <0.05 | 2.41 | 2.35 | 2.33 |
| 45°C | 2X | 2897 | 1074 | 348 | <0.005 | <0.005 | <0.005 | 2.53 | 2.49 | 2.36 |
| 45°C | 4X | 2673 | 519 | 144 | <0.005 | <0.005 | <0.005 | 2.45 | 2.38 | 2.36 |
| 45°C | 6X | 2426 | 201 | 10 | <0.005 | <0.005 | <0.005 | 2.50 | 2.43 | 2.41 |

### <Example 8>

### Method of preparing low molecular weight hyaluronic acid using SX-1G activated carbon

The reaction condition of <Example 1> was used except Norit@ SX-1G (Hereafter SX-1G)instead of CA1 activated carbon to perform comparative analysis of productivity of low molecular hyaluronic acid.

After 3, 6, and 18 hours, each sample was collected, and an endotoxin, a hyaluronic acid concentration, and an average molecular weight were analyzed according to the analysis method. The result is noted in [table 8]. At 35 °C, 6X for 6 hrs, 1,000,000 Da, and for 18 hrs 500,000 Da of hyaluronic acid was obtained. At 45 °C 2X for 18 hrs, 500,000 Da, 6x for 18 hrs, 10,000 Da of hyaluronic acid was obtained. The inventors confirmed that the endotoxin was less than 0.5 EU/mg and it was found that an increase of the reaction temperature is effective in further reduction of the endotoxin same as the result of the above results. Also, the concentration of hyaluronic acid was maintained at least 93% or more under all reaction conditions of SX-1G.

**Table 8**

| Result after reaction of hyaluronic acid with SX-1G activated carbon | | | | | | | | | | |
|---|---|---|---|---|---|---|---|---|---|---|
| Rxn Temp. | Conc. Of Acticated carbon | *MW, kDa* | | | Endotoxin, EU/mg | | | g HA/L | | |
| | | *3h* | *6h* | *18h* | 3h | 6h | 18h | 3h | 6h | 18h |
| 35°C | 2X | 3529 | 2408 | 1241 | <0.05 | <0.05 | <0.05 | 2.54 | 2.48 | 2.46 |
| 35°C | 4X | 3119 | 1540 | 757 | <0.05 | <0.05 | <0.05 | 2.50 | 2.47 | 2.43 |
| 35°C | 6X | 3005 | 1099 | 427 | <0.05 | <0.05 | <0.05 | 2.56 | 2.51 | 2.50 |
| 45°C | 2X | 3319 | 1315 | 471 | <0.005 | <0.005 | <0.005 | 2.52 | 2.48 | 2.36 |
| 45°C | 4X | 2971 | 636 | 196 | <0.005 | <0.005 | <0.005 | 2.51 | 2.47 | 2.46 |
| 45°C | 6X | 2696 | 247 | 14 | <0.005 | <0.005 | <0.005 | 2.50 | 2.48 | 2.40 |

### <Example 9>

### Method of preparing low molecular weight hyaluronic acid using SX-ULTRA activated carbon

The reaction condition of <Example 1> was used except Norit® SX-ULTRA (Hereafter SX-ULTRA) instead of CA1 activated carbon to perform comparative analysis of productivity of low molecular hyaluronic acid.

After 3, 6, and 18 hours, each sample was collected, and an endotoxin, a hyaluronic acid concentration, and an average molecular weight were analyzed according to the analysis method. The result is noted in [table 9]. At 25 °C, under a condition of SX-ULTRA 4X, for 18 hours, 1,000,000 Da of hyaluronic acid was obtained. At 35 °C 2X for 18 hrs, 1,000,000 Da of hyaluronic acid was obtained and 4x for 18 hrs, 500,000 Da of hyaluronic acid was obtained. At 45 °C 2X for 6 hrs, 1,000,000 Da, 4x for 6 hrs, 500,000 Da of hyaluronic acid was obtained and 6x for 18 hrs, 10,000 Da of hyaluronic acid was obtained. The inventors confirmed that the endotoxin was less than 0.5 EU/mg and it was found that an increase of the reaction temperature is effective in further reduction of the endotoxin same as the result of the above results. Also, the concentration of hyaluronic acid was maintained at least 93% or more under all reaction conditions of SX-ULTRA.

**Table 9**

| Result after reaction of hyaluronic acid with SX-ULTRA activated carbon | | | | | | | | | | |
|---|---|---|---|---|---|---|---|---|---|---|
| Rxn Temp. | Conc. Of Acticated carbon | *MW, kDa* | | | Endotoxin, EU/mg | | | g HA/L | | |
| | | *3h* | *6h* | *18h* | 3h | 6h | 18h | 3h | 6h | 18h |
| 25 °C | 2X | 3268 | 2951 | 1854 | <0.5 | <0.5 | <0.5 | 2.48 | 2.41 | 2.39 |
| 25 °C | 4X | 3171 | 1795 | 899 | <0.5 | <0.5 | <0.5 | 2.50 | 2.47 | 2.33 |
| 25 °C | 6X | 3092 | 1426 | 599 | <0.5 | <0.5 | <0.5 | 2.49 | 2.46 | 2.41 |
| 35 °C | 2X | 3176 | 1966 | 945 | <0.05 | <0.05 | <0.05 | 2.54 | 2.51 | 2.46 |
| 35 °C | 4X | 2779 | 1271 | 559 | <0.05 | <0.05 | <0.05 | 2.48 | 2.41 | 2.39 |
| 35 °C | 6X | 2704 | 897 | 322 | <0.05 | <0.05 | <0.05 | 2.41 | 2.35 | 2.33 |
| 45 °C | 2X | 2897 | 1074 | 348 | <0.005 | <0.005 | <0.005 | 2.53 | 2.49 | 2.36 |
| 45 °C | 4X | 2673 | 519 | 144 | <0.005 | <0.005 | <0.005 | 2.45 | 2.38 | 2.36 |
| 45 °C | 6X | 2426 | 201 | 10 | <0.005 | <0.005 | <0.005 | 2.50 | 2.43 | 2.41 |

### <Example 10>

### Method of preparing low molecular weight hyaluronic acid using PAC-200C activated carbon

The reaction condition of <Example 1> was used except Norit® PAC-200C (Hereafter PAC-200C)instead of CA1 activated carbon to perform comparative analysis of productivity of low molecular hyaluronic acid.

After 3, 6, and 18 hours, each sample was collected, and an endotoxin, a hyaluronic acid concentration, and an average molecular weight were analyzed according to the analysis method. The result is noted in [table 10]. At 35 °C 6X for 6 hrs, 1,000,000 Da of hyaluronic acid was obtained. At 45 °C 2X for 18 hrs, 500,000 Da, 4x for 6 hrs, 1,000,000 Da of hyaluronic acid was obtained and 6x for 18 hrs, 10,000 Da of hyaluronic acid was obtained. The inventors confirmed that the endotoxin was less than 0.5 EU/mg and it was found that an increase of the reaction temperature is effective in further reduction of the endotoxin same as the result of the above results. Also, the concentration of hyaluronic acid was maintained at least 93% or more under all reaction conditions of PAC-200C.

**Table 10**

| Result after reaction of hyaluronic acid with PAC-200C activated carbon | | | | | | | | | | |
|---|---|---|---|---|---|---|---|---|---|---|
| Rxn Temp. | Conc. Of Acticated carbon | *MW, kDa* | | | Endotoxin, EU/mg | | | g HA/L | | |
| | | *3h* | *6h* | *18h* | 3h | 6h | 18h | 3h | 6h | 18h |
| 35 °C | 2X | 3388 | 2311 | 1429 | <0.05 | <0.05 | <0.05 | 2.58 | 2.50 | 2.48 |
| 35 °C | 4X | 2988 | 1478 | 1279 | <0.05 | <0.05 | <0.05 | 2.54 | 2.49 | 2.39 |
| 35 °C | 6X | 2885 | 1100 | 721 | <0.05 | <0.05 | <0.05 | 2.60 | 2.53 | 2.52 |
| 45 °C | 2X | 3277 | 1893 | 542 | <0.005 | <0.005 | <0.005 | 2.48 | 2.46 | 2.37 |
| 45 °C | 4X | 2794 | 915 | 330 | <0.005 | <0.005 | <0.005 | 2.42 | 2.38 | 2.35 |
| 45 °C | 6X | 2589 | 355 | 15 | <0.005 | <0.005 | <0.005 | 2.50 | 2.38 | 2.33 |

### <Example 11>

### Method of preparing low molecular weight hyaluronic acid using DARCO KBB activated carbon

The reaction condition of <Example 1> was used except Norit® DARCO RBB (Hereafter PAC-200C)instead of CA1 activated carbon to perform comparative analysis of productivity of low molecular hyaluronic acid.

After 3, 6, and 18 hours, each sample was collected, and an endotoxin, a hyaluronic acid concentration, and an average molecular weight were analyzed according to the analysis method. The result is noted in [table 11]. At 25 °C, 4X for 18 hrs, 1,000,000 Da of hyaluronic acid was obtained. In a case where the reaction was carried out at 35 °C, the decomposition capability was highly increased and in case of 2x for 18 hrs, 1,000,000 Da, 4x for 18 hrs, 500,000 Da of hyaluronic acid was obtained. At 45 °C, 2x for 18 hrs, 500,000 Da, 4x for 6 hrs, 500,000 Da, 6X for 18 hrs, 10,000 Da of hyaluronic acid was obtained. The inventors confirmed that the endotoxin was less than 0.5 EU/mg and it was found that an increase of the reaction temperature is effective in further reduction of the endotoxin same as the result of the above results. Also, the concentration of hyaluronic acid was maintained at least 92% or more under all reaction conditions of DARCO KBB.

**Table 11**

| Result after reaction of hyaluronic acid with DARCO KBB activated carbon | | | | | | | | | | |
|---|---|---|---|---|---|---|---|---|---|---|
| Rxn Temp. | Conc. Of Acticated carbon | *MW, kDa* | | | Endotoxin, EU/mg | | | g HA/L | | |
| | | *3h* | *6h* | *18h* | 3h | 6h | 18h | 3h | 6h | 18h |
| 25 °C | 2X | 3380 | 2500 | 1454 | <0.5 | <0.5 | <0.5 | 2.50 | 2.47 | 2.32 |
| 25 °C | 4X | 3448 | 2173 | 954 | <0.5 | <0.5 | <0.5 | 2.53 | 2.50 | 2.45 |
| 25 °C | 6X | 3380 | 1426 | 410 | <0.5 | <0.5 | <0.5 | 2.52 | 2.39 | 2.39 |
| 35 °C | 2X | 3311 | 1966 | 916 | <0.05 | <0.05 | <0.05 | 2.54 | 2.49 | 2.35 |
| 35 °C | 4X | 3448 | 1454 | 463 | <0.05 | <0.05 | <0.05 | 2.45 | 2.43 | 2.39 |
| 35 °C | 6X | 3380 | 994 | 123 | <0.05 | <0.05 | <0.05 | 2.51 | 2.45 | 2.38 |
| 45 °C | 2X | 3380 | 881 | 492 | <0.005 | <0.005 | <0.005 | 2.52 | 2.49 | 2.34 |
| 45 °C | 4X | 3378 | 473 | 120 | <0.005 | <0.005 | <0.005 | 2.47 | 2.41 | 2.39 |
| 45 °C | 6X | 3346 | 15 | 9 | <0.005 | <0.005 | <0.005 | 2.51 | 2.44 | 2.40 |

### <Example 12>

### Method of preparing low molecular weight hyaluronic acid using DARCO A-51 activated carbon

The reaction condition of <Example 1> was used except Norit® DARCO A-51 (Hereafter PAC- A-51)instead of CA1 activated carbon to perform comparative analysis of productivity of low molecular hyaluronic acid.

After 3, 6, and 18 hours, each sample was collected, and an endotoxin, a hyaluronic acid concentration, and an average molecular weight were analyzed according to the analysis method. The result is noted in [table 12]. At 25 °C, 4X for 18 hrs, 1,000,000 Da of hyaluronic acid was obtained. In a case where the reaction was carried out at 35 °C, the decomposition capability was highly increased and in case of 6x for 6 hrs, 1,000,000 Da, for 18 hrs, 100,000 Da of hyaluronic acid was obtained. At 45 °C, 2x for 18 hrs, 500,000 Da, 4x for 6 hrs, 500,000 Da, 6X for 18 hrs, 10,000 Da of hyaluronic acid was obtained. The inventors confirmed that the endotoxin was less than 0.5 EU/mg and it was found that an increase of the reaction temperature is effective in further reduction of the endotoxin same as the result of the above results. Also, the concentration of hyaluronic acid was maintained at least 93% or more under all reaction conditions of DARCO A-51.

**Table 12**

| Result after reaction of hyaluronic acid with DARCO A-51 activated carbon | | | | | | | | | | |
|---|---|---|---|---|---|---|---|---|---|---|
| Rxn Temp. | Conc. Of Acticated carbon | *MW, kDa* | | | Endotoxin, EU/mg | | | g HA/L | | |
| | | 3h | *6h* | *18h* | 3h | 6h | 18h | 3h | 6h | 18h |
| 25 °C | 2X | 3554 | 2527 | 1499 | <0.5 | <0.5 | <0.5 | 2.54 | 2.53 | 2.42 |
| 25 °C | 4X | 3390 | 2217 | 994 | <0.5 | <0.5 | <0.5 | 2.54 | 2.50 | 2.48 |
| 25 °C | 6X | 3345 | 1412 | 497 | <0.5 | <0.5 | <0.5 | 2.52 | 2.45 | 2.40 |
| 35 °C | 2X | 3311 | 1965 | 898 | <0.05 | <0.05 | <0.05 | 2.54 | 2.46 | 2.41 |
| 35 °C | 4X | 3276 | 1439 | 477 | <0.05 | <0.05 | <0.05 | 2.53 | 2.51 | 2.45 |
| 35 °C | 6X | 3307 | 916 | 125 | <0.05 | <0.05 | <0.05 | 2.52 | 2.49 | 2.42 |
| 45 °C | 2X | 3207 | 757 | 496 | <0.005 | <0.005 | <0.005 | 2.49 | 2.43 | 2.34 |
| 45 °C | 4X | 3173 | 402 | 127 | <0.005 | <0.005 | <0.005 | 2.45 | 2.37 | 2.31 |
| 45 °C | 6X | 3138 | 16 | 10 | <0.005 | <0.005 | <0.005 | 2.50 | 2.35 | 2.34 |

### <Example 13>

### Method of preparing chicken's comb -derived low molecular weight hyaluronic acid

In the present Example, the high molecular weight hyaluronic acid raw material was extracted and purified from chicken's comb, and its average molecular weight was 3,500,000 Da.

In this experiment, CA1 activated carbon was selected as a molecular weight adjuster, and then the experimental result of chicken's comb -derived hyaluronic acid was compared to that of microorganism -derived hyaluronic acid. Hereinafter, the experiment will be described in detail.

The concentration of chicken's comb -derived hyaluronic acid raw material was adjusted to 2.5g HA/L. Then, the hyaluronic acid was introduced in an amount of 300 mℓ into a 1 L glass beaker, and CA1 activated carbon was introduced into a glass beaker at a concentration twice, 4 times and 6 times greater than hyaluronic acid concentration. Then, impeller (diameter 5 cm) made of teflon was rotated at a 300 rpm rate. In order to prevent mixing of impurities or evaporation of a reaction liquid to the maximum until the completion of the reaction, impeller portions other than the rotation portion of the impeller were sealed with a cover. Reaction temperatures were 25 °C.

The result of chicken's comb -derived hyaluronic acid as a control group was compared to that in <Example 1>. After 3, 6, and 18 hours, each sample was collected, and the change was analyzed. The change of chicken's comb -derived hyaluronic acid average molecular weight is noted in [table 13].

It was found that chicken's comb -derived hyaluronic acid was decomposed by CA1 activated carbon in the same manner as that in microorganism -derived hyaluronic acid of <Example 1>. This indicates that there is no difference in the molecular weight lowering process between microorganism -derived raw material and chicken's comb -derived raw material.

**Table 13**

| Changes of Molecular weight of chicken's comb -derived hyaluronic acid | | | | |
|---|---|---|---|---|
| Origin of hyaluronic acid | Conc. Of activated carbon | MW, kDa | | |
| | | 3h | 6h | 18h |
| microorganism | CA1 2X | 3519 | 2089 | 1485 |
| microorganism | CA1 4X | 3519 | 1485 | 994 |
| microorganism | CA1 6X | 3519 | 975 | 523 |
| chicken's comb | CA1 2X | 3381 | 1371 | 1015 |
| chicken's comb | CA1 4X | 3519 | 994 | 751 |
| chicken's comb | CA1 6X | 3314 | 653 | 455 |

### <Example 14>

### Method for producing hyaluronic acid having an average molecular weight of 2,000,000 Da in an industrial process

In a 1 ton fermenter, *Streptococcus* sp. ID9102(KCTC11395BP) was cultured under the same culture medium condition as described in <Example 1> so as to obtain 500 L culture. This culture was purified so as to produce a composition of 500 L. The average molecular weight of hyaluronic acid was 3,500,000 Da, the concentration of hyaluronic acid was 5 g/L, and the concentration of endotoxin was much greater than 5.0 EU/mg.

Activated carbon was selected as a molecular weight adjuster, and was introduced in an amount 4 times that of hyaluronic acid. The lowering reaction of molecular weight was carried out in a 500 L reactor coated with Teflon, and the reaction temperature was 45 °C. After 4 hours from the reaction, adsorbent was removed and the reaction was ended. After the reaction, a hyaluronic acid concentration of the resultant product, an endotoxin concentration, and an average molecular weight of hyaluronic acid were measured.

As a result, the average molecular weight of hyaluronic acid was 2,000,000 Da, the concentration of hyaluronic acid was 5 g/L, and the concentration of endotoxin was 0.005 EU/mg or less.

The hyaluronic acid filtrate whose molecular weight was lowered was precipitated and dried so as to provide 2 kg of hyaluronic acid having an average molecular weight of 2,000,000 Da.

### <Example 15>

### Method for producing hyaluronic acid having an average molecular weight of 1,000,000 Da in an industrial process

500 L of hyaluronic acid composition was prepared under the same condition as described in <Example 14>. The average molecular weight of hyaluronic acid was 3,500,000 Da, the concentration of hyaluronic acid was 5 g/L, and the concentration of endotoxin was much greater than 5.0 EU/mg.

The reaction condition of the activated carbon was the same in <Example 14>, and the reaction time was 6 hours. After 6 hours from the reaction, adsorbent was removed and the reaction was ended. After the reaction, a hyaluronic acid concentration of the resultant product, an endotoxin concentration, and an average molecular weight of hyaluronic acid were measured.

As a result, the average molecular weight of hyaluronic acid was 1,000,000 Da, the concentration of hyaluronic acid was 5 g/L, and the concentration of endotoxin was 0.005 EU/mg or less.

The hyaluronic acid filtrate whose molecular weight was lowered was precipitated and dried so as to provide 2 kg of hyaluronic acid having an average molecular weight of 1,000,000 Da.

### <Example 16>

### Method for producing hyaluronic acid having an average molecular weight of 500,000 Da in an industrial process

500 L of hyaluronic acid composition was prepared under the same condition as described in <Example 14>. The average molecular weight of hyaluronic acid was 3,500,000 Da, the concentration of hyaluronic acid was 5 g/L, and the concentration of endotoxin was much greater than 5.0 EU/mg.

The reaction condition of the activated carbon was the same in <Example 14>, and the reaction time was 7 hours. After 7 hours from the reaction, adsorbent was removed and the reaction was ended. After the reaction, a hyaluronic acid concentration of the resultant product, an endotoxin concentration, and an average molecular weight of hyaluronic acid were measured.

As a result, the average molecular weight of hyaluronic acid was 500,000 Da, the concentration of hyaluronic acid was 5 g/L, and the concentration of endotoxin was 0.005 EU/mg or less.

The hyaluronic acid filtrate whose molecular weight was lowered was precipitated and dried so as to provide 2 kg of hyaluronic acid having an average molecular weight of 500,000 Da.

### <Example 17>

### Method for producing hyaluronic acid having an average molecular weight of 100,000 Da in an industrial process

500 L of hyaluronic acid composition was prepared under the same condition as described in <Example 14>. The average molecular weight of hyaluronic acid was 3,500,000 Da, the concentration of hyaluronic acid was 5 g/L, and the concentration of endotoxin was much greater than 5.0 EU/mg.

The reaction condition of the activated carbon was the same in <Example 14>, and the reaction time was 8 hours. After 8 hours from the reaction, adsorbent was removed and the reaction was ended. After the reaction, a hyaluronic acid concentration of the resultant product, an endotoxin concentration, and an average molecular weight of hyaluronic acid were measured.

As a result, the average molecular weight of hyaluronic acid was 100,000 Da, the concentration of hyaluronic acid was 5 g/L, and the concentration of endotoxin was 0.005 EU/mg or less.

The hyaluronic acid filtrate whose molecular weight was lowered was precipitated and dried so as to provide 2 kg of hyaluronic acid having an average molecular weight of 100,000 Da.

Through <Example 14> to <Example 17>, the present inventors developed a method for producing high quality of low molecular weight hyaluronic acid, which is easy to apply to industry.

### Industrial Applicability

The method of the present invention for lowering a molecular weight of high molecular hyaluronic acid has a convenient process without the inconvenience of reprocessing for the removal of input materials, or requiring pH treatment, various reaction catalysts, and complicated additional treatment conditions (such as heat treatment) as in a conventional method. Furthermore, the method has an effect for removing impurities. Thus, through the method, it is possible to conveniently and economically produce low molecular weight hyaluronic acid with high purity. The inventive method for lowering the molecular weight has an advantage in that according to a change of a reaction condition using activated carbon, the molecular weight of low molecular weight hyaluronic acid can be variously adjusted. The low molecular weight hyaluronic acid produced by the method of the present invention can be produced in accordance with the standards of medical supplies while the hyaluronic acid's own characteristic can be maintained. Also, it can be produced in accordance with the standards of cosmetics or foods.

## Claims

1. A method for preparing low molecular weight hyaluronic acid comprising the step of bringing hyaluronic acid into contact with activated carbon, wherein the activated carbon is used as reaction medium in order to convert high molecular weight hyaluronic acid into low molecular weight hyaluronic acid having a predetermined molecular weight, and
wherein the high molecular weight hyaluronic acid is obtained through microorganism culture or chicken's comb extraction, and
wherein the ratio of hyaluronic acid to activated carbon is 1:2 to 1:6.

2. The method of claim 1, wherein the temperature for bringing hyaluronic acid into contact with activated carbon is 25 °C to 45 °C, and the time for bringing hyaluronic acid into contact with activated carbon is 3 hrs to 18 hrs.

## Patentansprüche

1. Verfahren zur Herstellung von Hyaluronsäure mit niedrigem Molekulargewicht, umfassend den Schritt des Inkontaktbringens von Hyaluronsäure mit Aktivkohle, wobei die Aktivkohle als Reaktionsmedium verwendet wird, um hochmolekulare Hyaluronsäure in niedermolekulare Hyaluronsäure mit einem vorbestimmten Molekulargewicht umzuwandeln, und
wobei hochmolekulare Hyaluronsäure durch Mikroorganismenkultur oder Hühnerkamm-Extraktion gewonnen wird, und
wobei das Verhältnis von Hyaluronsäure zu Aktivkohle 1:2 bis 1:6 beträgt.

2. Verfahren nach Anspruch 1, wobei die Temperatur zum Inkontaktbringen von Hyaluronsäure mit Aktivkohle 25 °C bis 45 °C beträgt und die Zeit zum Inkontaktbringen von Hyaluronsäure mit Aktivkohle 3 h bis 18 h beträgt.

## Revendications

1. Procédé de préparation d'acide hyaluronique de bas poids moléculaire comprenant l'étape de mise en contact de l'acide hyaluronique avec du charbon activé, le charbon activé étant utilisé comme milieu réactionnel pour convertir l'acide hyaluronique de haut poids moléculaire en acide hyaluronique de bas poids moléculaire ayant un poids moléculaire prédéterminé, et
dans lequel l'acide hyaluronique de haut poids moléculaire est obtenu par culture de micro-organismes ou extraction du peigne de poulet, et
dans lequel le rapport de l'acide hyaluronique au charbon activé est de 1:2 à 1:6.

2. Procédé selon la revendication 1, dans lequel la température de mise en contact de l'acide hyaluronique avec le charbon activé est de 25 °C à 45 °C, et le temps de mise en contact de l'acide hyaluronique avec le charbon activé est de 3h à 18h.
